# EUROPEAN PATENT SPECIFICATION

(11) **EP 3 337 494 B1**
(45) Date of publication and mention of the grant of the patent: **11.03.2020**
(21) Application number: 16757590.1
(22) Date of filing: 16.08.2016
(51) Int. Cl.: A61K 38/12, C07K 7/54, A61P 31/04, A61P 15/14, A61P 15/00, C07K 7/56

(54) **LYSOBACTIN FOR USE IN THE TREATMENT OF BOVINE MASTITIS**
LYSOBACTIN ZUR BEHANDLUNG VON RINDERMASTITIS
LYSOBACTINE POUR UNE UTILISATION DANS LE TRAITEMENT DE LA MASTITE BOVINE

(30) Priority: 17.08.2015 EP 15181209
(43) Date of publication of application: 27.06.2018
(73) Proprietor: Bayer Animal Health GmbH, 51373 Leverkusen (DE)
(72) Inventor: SCHIFFER, Guido, 42111 Wuppertal (DE); FÄLKER, Stefan, 50676 Köln (DE); DAUBE, Gert, 51766 Engelskirchen (DE); FRAATZ, Kristine, 51399 Burscheid (DE); WIEHL, Wolfgang, 50999 Köln (DE); KÖBBERLING, Johannes, 41466 Neuss (DE)
(74) Representative: Tier 1 Patents
(86) International application number: PCT/EP2016/069380
(87) International publication number: WO 2017/029271

(56) References cited:
- US-A- 4 754 018
- US-A1- 2005 075 281
- US-A1- 2006 264 358
- US-A1- 2008 051 424
- US-A1- 2008 058 251
- US-A1- 2008 058 253
- US-A1- 2009 203 582
- BONNER D P ET AL: "LYSOBACTIN A NOVEL ANTIBACTERIAL AGENT PRODUCED BY LYSOBACTER-SP II. BIOLOGICAL PROPERTIES", 1988, JOURNAL OF ANTIBIOTICS (TOKYO), VOL. 41, NR. 12, PAGE(S) 1745-1751, XP002745774, ISSN: 0021-8820 abstract "Materials and Methods" on pages 1745-1746
- O'SULLIVAN J ET AL: "LYSOBACTIN, A NOVEL ANTIBACTERIAL AGENT PRODUCED BY LYSOBACTER SP. I. TAXONOMY, ISOLATION AND PARTIAL CHARACTERIZATION", JOURNAL OF ANTIBIOTICS, JAPAN ANTIBIOTICS RESEARCH ASSOCIATION, TOKYO, JP, no. 12, December 1988 (1988-12), pages 1740-1744, XP009059528, ISSN: 0021-8820

## Description

The present invention relates to lysobactin for use in the treatment of bovine mastitis.

Mastitis is the inflammation of udder tissue and continues to be the most frequent and costly disease of dairy cattle. Financial losses due to mastitis occur for both subclinical and clinical stages of the disease. Losses caused by subclinical mastitis are well documented. Each doubling of the somatic cell count (SCC) above 50,000 cells/ml results in a loss of 0.4 kg and 0.6 kg of milk per day in first lactation and older cows, respectively (Hortet P, H. Seegers. 1998. Calculated milk production losses associated with elevated somatic cell counts in dairy cows: review and critical discussion. Vet Res. 29(6):497-510). Losses caused by clinical mastitis include discarded milk, transient reductions in milk yield and premature culling. Treatment of mastitis should be targeted towards the causative bacteria whenever possible.

Pyörälä S in Irish Veterinary Journal, Volume 62 Supplement 40-44 2009 has the following suggestions for antimicrobial treatment of clinical mastitis due to different pathogens:

| **Microorganism** | **Species** | **Drug of choice** | **Alternative** |
|---|---|---|---|
| Streptococci | *Streptococcus agalacticae* | Penicillin G | |
| | *Streptococcus dysgalacticae* | | |
| | *Streptococcus uberis* | | |
| | *Enterococci* | According to susceptibility testing | |
| Staphylococci | *Staphylococcus aureus* | Penicillin G | |
| | Coagulase negative staphylococci β-lactamase -ve | | |
| | *Staphylococcus aureus* | No antimicrobials | Cloxacillin Macrolides Lincosamides |
| | Coagulase negative staphylococci β-lactamase +ve | | |
| Coliforms | *Escherichia coli* | No antimicrobials | Fluoroquinolones Cephalosporins |
| | *Klebsiella spp.* | | |

A recent pharmaceutical composition for the treatment of mastitis in dairy cows is Ubrolexin®, a broad spectrum antibiotic against both gram positive and gram negative mastitis-causing bacteria. Ubrolexin® is a combination of cefalexin and kanamycin and is indicated for the treatment of clinical mastitis due to *Staphylococcus aureus, Streptococcus dysgalacticae, Streptococcus uberis* and *Escherichia coli.* The mode of action for cefalexin is an irreversible binding to D-alanine transpeptidase which disrupts the bacterial cell wall synthesis. Kanamycin acts by attaching to the 30S subunit of membrane associated ribosomes and inhibits bacterial protein synthesis.

A reoccurring problem with antibiotics in the prior art is that the pathogens may develop resistance towards the drugs employed in the treatment of the diseases they cause. Therefore, new active pharmaceutical ingredients are constantly sought after.

Lysobactin was first isolated from the fermentation broth of *Lysobacter sp.* SC-14076 (ATCC 53042). Independently, scientists discovered katanosin A and B from a soil bacterium related to the genus Cytophaga (producer strain PBJ-5356). Katanosin B turned out to be identical to lysobactin. Bacterial cell wall biosynthesis is the primary target of these antibiotics; however, the mechanism of action is different from that of vancomycin. The lysobactins are cyclic depsipetides containing several nonproteinogenic D- and L-amino acids.

The natural product lysobactin and some derivatives are described as having antibacterial activity in US 4,754,018. The isolation and antibacterial activity of lysobactin is also described in EP 196 042 A1 and JP 01-132600.

The antibacterial activity of lysobactin and katanosin A is furthermore described in O'Sullivan, J. et al., J. Antibiot. (1988) 41 :1740-1744, Bonner, D. P. et al., J. Antibiot. (1988) 41:1745-1751, Shoji, J. et al., J. Antibiot. (1988) 41:713-718 and Tymiak, A. A. et al., J. Org. Chem. (1989) 54:1149-1157.

In contrast to the modes of action for cefalexin and kanamycin, lysobactin has been suggested to directly interact with the bacterial cell wall precursor lipid II (Breukink E. and de Kruijff B., Nat. Rev. Drug Disc. (2006) 5:321-323).

Derivatives of lysobactin are the subject of the patent applications WO 2004/099239 A1, WO 2006/042653 A1, WO 2006/042654 A1, WO 2006/042655 A1, WO 2006/048139 A1, WO 2006/048140 A1, WO 2006/048156 A1, WO 2007/085456 A1, WO 2007/118691 A1 and WO 2007/118693 A1.

None of the prior art is concerned with the treatment of bovine mastitis using lysobactin.

There is a need in the art for a bovine mastitis treatment that does not suffer from the drawback of bacterial resistance towards the active ingredient. The present invention has the object of providing an agent for such a treatment.

Accordingly, the present invention is directed towards lysobactin for use in the treatment of bovine mastitis.

In the context of the present invention, the term "lysobactin" refers to the substance with the CAS number 118374-47-3 and its physiologically acceptable salts. Lysobactin has the following structure:

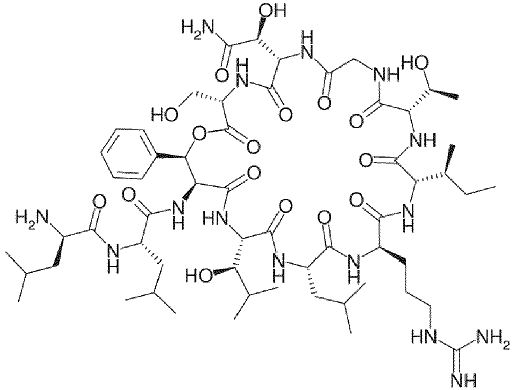

The bovine mastitis to be addressed is in particular the mastitis of dairy cattle.

Physiologically acceptable salts of lysobactin include acid addition salts of mineral acids, carboxylic acids and sulphonic acids, e.g. salts of hydrochloric acid, hydrobromic acid, sulphuric acid, phosphoric acid, methanesulphonic acid, ethanesulphonic acid, toluenesulphonic acid, benzenesulphonic acid, naphthalenedisulphonic acid, acetic acid, trifluoroacetic acid, propionic acid, lactic acid, tartaric acid, malic acid, citric acid, fumaric acid, maleic acid and benzoic acid.

Physiologically acceptable salts of lysobactin also include salts of conventional bases such as, by way of example and preferably, alkali metal salts (e.g. sodium and potassium salts), alkaline earth metal salts (e.g. calcium and magnesium salts) and ammonium salts derived from ammonia or organic amines having 1 to 16 C atoms, such as, by way of example and preferably, ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, arginine, lysine, ethylenediamine and N-methylpiperidine.

Lysobactin can act systemically and/or locally. For this purpose, it can be administered in a suitable way such as, for example, by the oral, parenteral, pulmonary, nasal, sublingual, lingual, buccal, rectal, intraauricular, dermal or transdermal route, or as implant or stent.

Lysobactin can be administered in administration forms suitable for these administration routes.

Suitable for oral administration are administration forms which function according to the prior art and deliver lysobactin rapidly and/or in modified fashion, and which contain lysobactin in crystalline and/or amorphized and/or dissolved form, such as, for example, tablets (uncoated or coated tablets, for example having enteric coatings or coatings which are insoluble or dissolve with a delay and control the release of lysobactin), tablets which disintegrate rapidly in the mouth, or films/wafers, films/lyophilizates, capsules (for example hard or soft gelatin capsules), sugar-coated tablets, granules, pellets, powders, emulsions, suspensions, aerosols or solutions.

Parenteral administration can take place with avoidance of an absorption step (e.g. intramammary, intravenous, intraarterial, intracardiac, intraspinal or intralumbar) or with inclusion of absorption (e.g. intramuscular, subcutaneous, intracutaneous, percutaneous or intraperitoneal). Administration forms suitable for parenteral administration are, inter alia, preparations for injection and infusion in the form of solutions, suspensions, emulsions, lyophilizates or sterile powders.

Suitable for the other administration routes are, for example, pharmaceutical forms for aqueous suspensions (lotions, shaking mixtures), lipophilic suspensions, ointments, creams, transdermal therapeutic systems (such as, for example, patches), milk, pastes, foams, dusting powders, implants or stents.

Lysobactin can be converted into the stated administration forms. This can take place in a manner known per se by mixing with inert, nontoxic, pharmaceutically suitable excipients. These excipients include, inter alia, carriers (for example microcrystalline cellulose, lactose, mannitol), solvents (e.g. liquid polyethylene glycols), emulsifiers and dispersants or wetting agents (for example sodium dodecyl sulphate, polyoxysorbitan oleate), binders (for example polyvinylpyrrolidone), synthetic and natural polymers (for example albumin), stabilizers (e.g. antioxidants such as, for example, ascorbic acid), colours (e.g. inorganic pigments such as, for example, iron oxides) and masking flavours and/or odours.

The present invention will be further described with reference to the following aspects and embodiments. They may be combined freely unless the context clearly indicates otherwise.

One embodiment concerns lysobactin for use in the treatment of bovine mastitis, wherein lysobactin is administered intramammarily.

Another embodiment concerns lysobactin for use in the treatment of bovine mastitis, wherein the bovine mastitis is a clinically manifest bovine mastitis.

Another embodiment concerns lysobactin for use in the treatment of bovine mastitis, wherein the bovine mastitis is a subclinical bovine mastitis.

Another embodiment concerns lysobactin for use in the treatment of bovine mastitis, wherein the lysobactin is provided at a dose of 25 to 1000 mg per udder quarter, preferably at a dose of 50 to 500 mg per udder quarter and more preferably at a dose of 100 to 300 mg per udder quarter.

It may nevertheless be necessary where appropriate to deviate from the stated amounts; in particular as a function of the bodyweight, route of administration, individual response to the active ingredient, nature of the preparation and time or interval over which administration takes place. Thus, it may be sufficient in some cases to make do with less than the aforementioned minimum amount, whereas in other cases the stated upper limit must be exceeded. It may in the event of administration of larger amounts be advisable to divide these into a plurality of individual doses over the day.

Another embodiment concerns lysobactin for use in the treatment of bovine mastitis, wherein the bovine mastitis is caused by *Staphylococcus* bacteria, *Streptococcus* bacteria, *Trueperella* bacteria and/or *Corynebacterium* bacteria. In particular, the bovine mastitis may be caused by *Staphylococcus aureus,* coagulase-negative staphylococci, *Streptococcus uberis, Streptococcus dysgalacticae* and/or *Streptococcus agalacticae.* Furthermore, the bovine mastitis may be caused by *Trueperella pyogenes.* Also, the bovine mastitis may be caused by *Corynebacterium bovis.* These microorganisms are the most common pathogens found in cases of bovine mastitis and that were found to be particular susceptible to a treatment with lysobactin according to the present invention.

Another aspect of the present invention is a pharmaceutical composition formulated for intramammary administration into bovine mammaries, wherein the composition comprises lysobactin. Preferably, the composition further comprises a pharmaceutically acceptable excipient and in particular a pharmaceutically acceptable carrier.

These excipients include, inter alia, carriers (for example microcrystalline cellulose, lactose, mannitol), solvents (e.g. liquid polyethylene glycols), emulsifiers and dispersants or wetting agents (for example sodium dodecyl sulphate, polyoxysorbitan oleate), binders (for example polyvinylpyrrolidone), synthetic and natural polymers (for example albumin), stabilizers (e.g. antioxidants such as, for example, ascorbic acid), colours (e.g. inorganic pigments such as, for example, iron oxides) and masking flavours and/or odours.

Another aspect of the present invention is the use of lysobactin for the preparation of pharmaceuticals for the treatment of bovine mastitis.

In one embodiment of the use according to the invention the bovine mastitis is a clinically manifest bovine mastitis.

In another embodiment of the use according to the invention the bovine mastitis is a subclinical bovine mastitis.

In another embodiment of the use according to the invention the bovine mastitis is caused by *Staphylococcus* bacteria, *Streptococcus* bacteria, *Trueperella* bacteria and/or *Corynebacterium* bacteria.

In particular, the bovine mastitis may be caused by *Staphylococcus aureus,* coagulase-negative staphylococci, *Streptococcus uberis, Streptococcus dysgalacticae* and/or *Streptococcus agalacticae.* Furthermore, the bovine mastitis may be caused by *Trueperella pyogenes.* Also, the bovine mastitis may be caused by *Corynebacterium bovis.*

The present invention further relates to a method of treating bovine mastitis, the method comprising the step of administering to a cow in need thereof a therapeutically effective amount of lysobactin.

### Examples:

The present invention will be further elucidated with reference to the following examples and figures without being limited to them.
- FIG. 1: shows the kill kinetics of lysobactin against *Staphylococcus aureus* and *Streptococcus uberis* (example 2)
- FIG. 2: shows the MIC changes of lysobactin against *Staphylococcus aureus* and *Streptococcus uberis* during serial passaging (example 4)
- FIG. 3: shows the efficacy of lysobactin in a *Staphylococcus aureus* acute mouse mastitis model (example 5)
- FIG. 4: shows the efficacy of lysobactin in a *Streptococcus uberis* challenge mouse mastitis model (example 6)
- FIG. 5: shows the concentration-time profile of lysobactin in milk after intramammary (IMAM) application to lactating Holstein cows (example 7)

### Example 1: In vitro antibacterial activity against mastitis pathogens

The in vitro antibacterial activity of lysobactin against common mastitis pathogens such as *Staphylococcus aureus,* Coagulase-negative staphylococci, *Streptococcus uberis, Streptococcus dysgalactiae, Streptococcus agalactiae, Trueperella pyogenes, Escherichia coli,* or *Klebsiella pneumoniae* was assessed by microbroth dilution MIC methodology as described by the Clinical and Laboratory Standards Institute (CLSI) in order to obtain the Minimal Inhibitory Concentration (MIC), which is defined as the lowest concentration of a substance that prevents visible growth of a bacterium. The results expressed as MIC₉₀ are summarized in the following table, where the MIC₉₀ is defined as the concentration at which the growth of at least 90% of the strains of a given species is inhibited.

| **Tested bacteria** | **MIC₉₀ [µg/ml] of lysobactin** |
|---|---|
| *Staphylococcus aureus* | 0.5 |
| Coagulase-negative staphylococci | 0.5 |
| *Streptococcus uberis* | 0.125 |
| *Streptococcus dysgalactiae* | 0.25 |
| *Streptococcus agalactiae* | 0.25 |
| *Trueperella pyogenes* | 0.5 |
| *Escherichia coli* | >16 |
| *Klebsiella pneumoniae* | >16 |

### Example 2: In vitro kill kinetics for mastitis pathogens

In order to assess the ability of lysobactin to kill bacteria in milk, flasks containing different concentrations of lysobactin in store-bought full-fat milk were inoculated with 1-2 x 10⁶ colony forming units/ml of a representative strain of either *Staphyloccus aureus* or *Streptococcus uberis.* The flasks were incubated for 24-48 hours in a shaking water bath at 35 +/- 2°C, and viable bacterial counts in each flask were determined at several time-points by diluting and plating samples on agar plates. A reduction of the number of viable bacteria in the initial inoculum by at least 99.9% is defined as bactericidal activity.

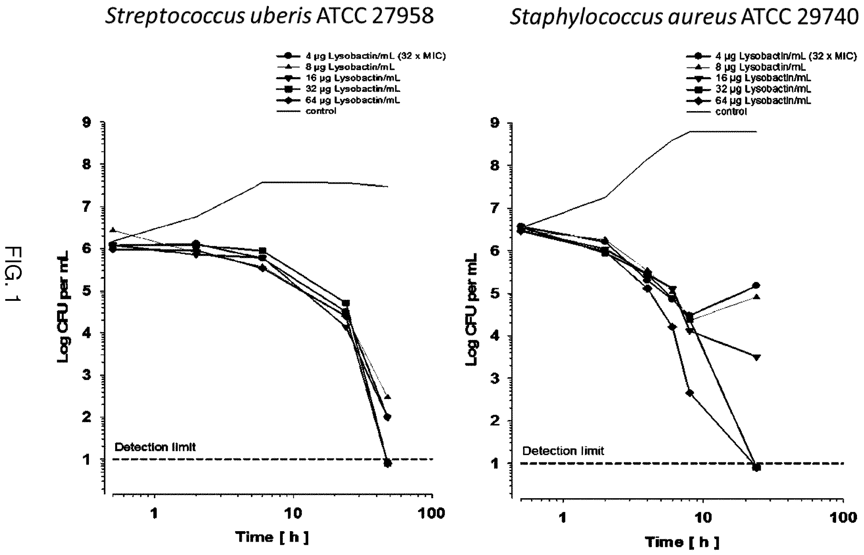

The kill kinetics of lysobactin against *Staphylococcus aureus* ATCC 29740 and *Streptococcus uberis* ATCC 27958 in milk were determined for concentrations of lysobactin of 4, 8, 16, 32 and 64 µg/mL. Results are depicted in FIG. 1 (CFU: colony forming units). A cidality at 24 h and 48 h, respectively, can be postulated for *S. aureus* and *S. uberis.*

### Example 3: In vitro assessment of spontaneous resistance development:

The frequency of spontaneous resistance development was assessed by plating at least 1x10⁹ colony forming units of the respective bacterial strain on agar plates containing lysobactin at either 4x or 8x the MIC and incubating the plates at 35 +/- 2°C. After 48 h, the number of colonies that had grown on the plates at lysobactin concentration above the MIC was divided by the number of bacteria that was initially plated. The resulting number is defined as the spontaneous resistance frequency, and is an indication for the likelihood of resistant isolates to appear during an infection.

As a conclusion, lysobactin at 4- and 8-fold MIC displays a very good resistance profile: no resistant isolates were detectable. The results are summarized in the following tables:

**Staphylococcus aureus ATCC 29740:**

| **Lysobactin concentration** | **plated CFU** | **24 h** | | **48 h** | |
|---|---|---|---|---|---|
| | | **obtained colonies** | **frequency of resistance** | **obtained colonies** | **frequency of resistance** |
| 2 µg/ml (4x MIC) | 3.52 x 10⁹ | 0 | < 2.84 x 10⁻¹⁰ | 0 | < 2.84 x 10⁻¹⁰ |
| 4 µg/ml (8x MIC) | 3.52 x 10⁹ | 0 | < 2.84 x 10⁻¹⁰ | 0 | < 2.84 x 10⁻¹⁰ |

**Streptococcus uberis ATCC 27958:**

| **Lysobactin concentration** | **plated CFU** | **24 h** | | **48 h** | |
|---|---|---|---|---|---|
| | | **obtained colonies** | **frequency of resistance** | **obtained colonies** | **frequency of resistance** |
| 2 µg/ml (4x MIC) | 2.4 x 10¹⁰ | 0 | < 4.17 x 10⁻¹¹ | 0 | <4.17 x 10⁻¹¹ |
| 4 µg/ml(8x MIC) | 2.4 x 10¹⁰ | 0 | < 4.17 x 10⁻¹¹ | 0 | < 4.17 x 10⁻¹¹ |

### Example 4: In vitro assessment of resistance development during serial passaging

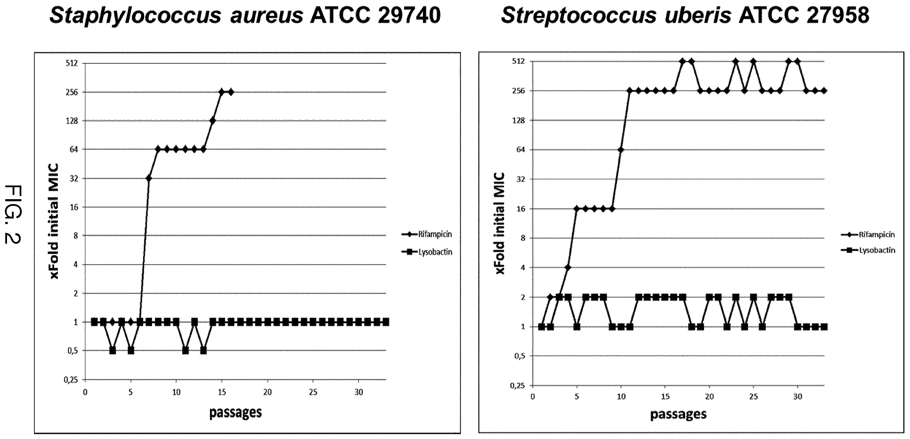

The appearance of MIC changes during constant exposure of bacteria to sub-MIC concentrations of lysobactin was assessed by serial passaging experiments. On the first day the MICs of *S. aureus* and *S. uberis* were assessed by microbroth dilution MIC methodology as described by the Clinical and Laboratory Standards Institute (CLSI). Each day for the following 33 consecutive days, bacteria from the well containing the highest concentrations allowing full growth were collected and used to inoculate new 96-well plates and to assess the MIC during constant exposure to lysobactin. The MIC obtained on each day was plotted over time. The results are shown in FIG. 2. Rifampicin, which is known to result in quick changes of the MIC, was used as positive control.

The results indicate a very good profile of lysobactin for resistance development during constant exposure, as the MICs for *S. aureus* and *S. uberis* remain constant over the 33 days period.

### Example 5: Efficacy in an acute mouse mastitis model with S. aureus

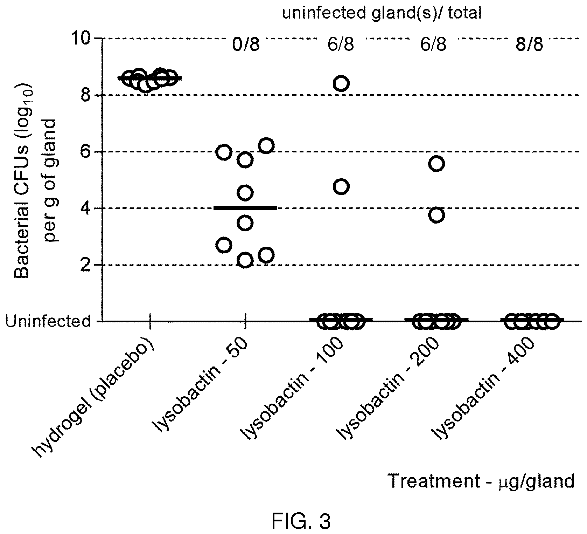

The efficacy of lysobactin (formulated in a hydrogel at pH 4.7) was tested in a *Staphylococcus aureus* acute mouse mastitis model established at the University of Sherbrooke, Canada (Broui-llette et al, Vet. Microbiol. (2004) 4:253-262), that is hereby incorporated by reference. Both the abdominal mammary glands (L4 and R4) of lactating CD-1 mice were intramammarily infected with 100 CFU (colony forming units) of *S. aureus.* The mice were treated intramammarily with lysobactin four hours after infection. Each treatment group contained at least 3 mice (6 quarters) 14 hours later (18 hours after inoculation) mice were sacrificed, mammary glands were harvested and the CFU content evaluated by plating 10-fold serial dilutions of mammary gland homogenates. The CFU content was expressed as log₁₀ count. The detection limit was 200 CFU/g of gland. Glands with less than 200 CFU/g were regarded as cleared. The results are shown in FIG. 3.

Intramammary instillation of 50 µg lysobactin reduces the median CFU content by ca. 4 logio, 400 µg lysobactin eliminates the infection from all infected glands.

### Example 6: Efficacy in an acute mouse mastitis model with S. uberis

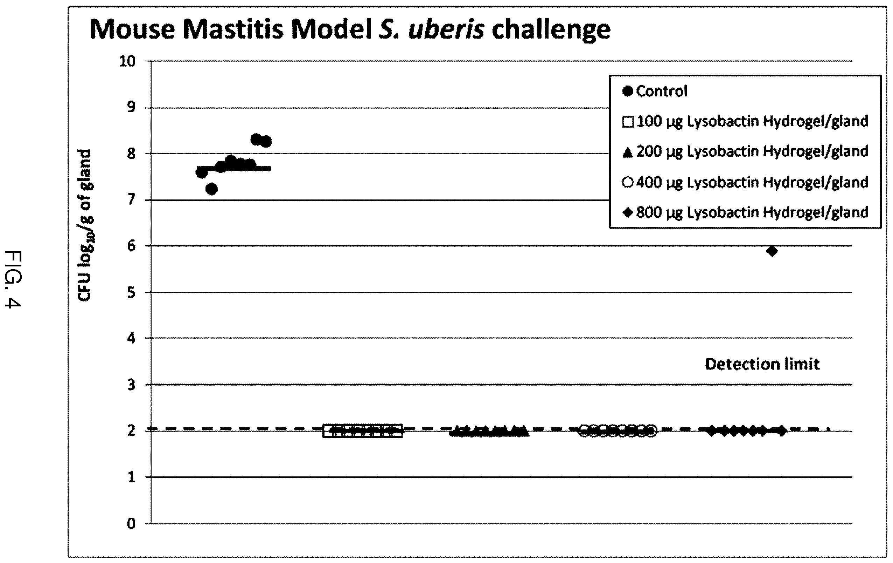

The efficacy of lysobactin (formulated in a hydrogel at pH 4.7) was tested in a *Streptococcus uberis* challenge mouse mastitis model. The results are shown in FIG. 4.

Twenty lactating mice were experimentally infected on the fourth pair of mammary glands with *S*. *uberis* around 12-15 days after birth of the offspring. Four hours after inoculation, groups of four mice were treated on the same glands with lysobactin at 100, 200, 400, or 800 µg/ gland formulated as hydrogel. The fifth group was treated solely with the hydrogel vehicle as negative control. Eighteen hours after infection the animals were euthanized, the glands were removed, homogenized, and bacterial colony forming units (CFUs) were determined by established microbiological methods. Subsequently CFU/mL homogenate as well as CFU/g of gland were calculated. The detection limit was approximately 100 CFU/g of gland. Antimicrobial activity of lysobactin against *Strep uberis* was determined by comparison of the mean CFUs/gland of the different dosing groups and the negative control group.

Glands of all animals of the control group showed an optimum infection rate (≥10⁷ CFU/g of gland) 18 hours after infection. With one exception in the highest dosing group all glands in the lysobactin treated groups had bacterial counts below the limit of detection (10² CFU/g). It can be concluded that lysobactin formulated as hydrogel has outstanding antibacterial efficacy against *S. uberis* at intramammary doses between 100 and 800 µg/gland.

### Example 7: Lysobactin in vivo cattle data - milk pharmacokinetic study in lactating Holstein cows

The study was designed as a non-pivotal study suitable to investigate the milk pharmacokinetics of the active substance lysobactin after single intracisternal application to lactating dairy cows.

The active substance was provided in a paraffine based service formulation suitable for intracisternal application containing 150 mg lysobactin B in 10 g oily suspension.

The test item was administered as single intracisternal treatment at a dose rate of 150 mg lysobactin to a single hind quarter of four lactating dairy cows each.

The dairy cows on study (Holstein cows) represented the target population in age, lactation number, lactation stage, milk yield and breed. The animals were stalled in a tie-barn and were fed with standard feed for dairy cows consisting of corn and gras silage and milk performance feed. Milking was twice daily at a 12 hour interval using a bucket-type milking device.

Frequent milk sampling was performed from the treated and respective control quarters prior to (0 h) and over a period of 168 h after treatment (0.5, 1, 2, 4, 6, 8, 12, 24, 36, 48, 60, 72, 84, 96, 120, 144, and 168 h) by manual stripping of the respective udder quarters. Milk samples at routine milking times were gathered prior to milking.

Concentrations of the active substance lysobactin in milk were analyzed by HPLC with detection by tandem mass spectrometry. The limit of quantitation was 0.05 mg/L.

Pharmacokinetic evaluation of milk concentration data was based on non-compartmental methods and comprised PK-parameters to adequately describe the absorption, distribution and elimination profile of the active substance in milk.

Summarized results derived from the treated udder quarters are presented in the following table. No lysobactin was detected in the control samples (untreated udder quarters).

**Mean milk pharmacokinetic results of lysobactin after single treatment**

| Matrix | Cₘₐₓ¹ | Tₘₐₓ² | t_{1/2}¹ | AUC_{inf}¹ | AUC₀₋₁₂ₕ¹ | AUC₀₋₂₄ₕ¹ |
|---|---|---|---|---|---|---|
| | mg/L | h | h | mg^{∗}h/L | mg^{∗}h/L | mg^{∗}h/L |
| Milk | 342 | 4 | 11.6 | 2321 | 1870 | 2213 |

| | | | | | | |
|---|---|---|---|---|---|---|
| *Dose applied to 1 quarter per cow was 150 mg lysobactin;* *Means are given as 1) geometric mean, 2) median* | | | | | | |

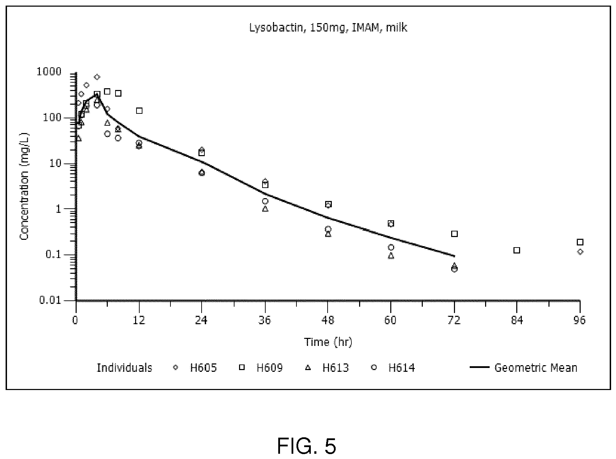

The concentration time curve of lysobactin is depicted in FIG. 5.

### Example 8: Lysobactin in vivo cattle data - dairy cow udder infection model with S. aureus

Fifteen healthy lactating dairy cows were experimentally infected with the mastitis pathogen *Staphylococcus aureus* on all four udder quarters. As soon as an udder quarter showed clinical symptoms of mastitis, such as swelling, pain, abnormal milk consistency, it was treated with either Lysobactin paraffine based ointment at two different concentrations, or Ubrolexin® (Cephalexin + Kanamycin, Boehringer Ingelheim), or saline solution as negative control. Treatments were randomly assigned to 42 udder quarters in total, either 50 mg lysobactin per quarter (in 11 quarters), or 150 mg Lysobactin (in 11 quarters), or Ubrolexin® (in 9 quarters), or saline solution (in 11 quarters). Only udder quarters that were bacteriologically positive for the challenge organism immediately prior to treatment (n=42) were eligible for assessment of microbiological and clinical cure. The diseased quarters were treated with these intramammary formulations two times with an interval of 24 hours in between. Udders were clinically examined and milk samples were taken before treatment and several times after that until three weeks after the second administration. Milk was inspected visually for deviation in its consistency and samples were evaluated for presence of the challenge organism and for somatic cell count to confirm the diagnosis mastitis. Diseased udder quarters were considered microbiologically cured when *Staphylococcus aureus* found in milk samples shortly before treatment could not be isolated from any milk sample taken within the time period between the third and the twenty-first day after the second treatment. Clinical cure was achieved when the local symptoms of mastitis had completely disappeared, and recovery of somatic cell counts (SCC) as parameter of udder inflammation was attained when all counts remained below 500.000 cells per mL of milk in the period mentioned above.

The microbiological cure rate on Day 3 and Day 21 after second treatment was 73% and 27% for Lysobactin 50 mg, 73% and 45% for Lysobactin 150 mg, 78% and 44% for Ubrolexin®, and 0% and 0% for saline solution, respectively. The local clinical cure rate on Day 3 and Day 21 after second treatment was 45% and 73% for Lysobactin 50 mg, 37% and 82% for Lysobactin 150 mg, 33% and 67% for Ubrolexin®, and 55% and 73% for saline solution, respectively. The SCC recovery rate on Day 3 and Day 21 after second treatment was 36% and 45% for Lysobactin 50 mg, 36% and 91% for Lysobactin 150 mg, 56% and 67% for Ubrolexin®, and 36% and 27% for saline solution, respectively.

It can be concluded that Lysobactin 150 mg shows similar or even better efficacy in comparison to the positive control product Ubrolexin® and superiority to Lysobactin 50 mg and Saline solution in the parameters microbiological and clinical cure, and SCC recovery.

### Example 9: Lysobactin in vivo cattle data - dairy cow udder infection model with S. uberis

Seventy-two healthy lactating dairy cows were experimentally infected with the mastitis pathogen *Streptococcus uberis* on two udder quarters per cow. As soon as an udder quarter showed clinical symptoms of mastitis (e.g. heat, swelling, redness, pain, abnormal milk consistency), it was treated with either lysobactin paraffine based ointment at two different concentrations, or Ubrolexin® (cephalexin + kanamycin, Boehringer Ingelheim).

Treatments were randomly assigned to 41 (clinical) or 37 (microbiological) udder quarters in total, either 50 mg lysobactin per quarter (in 15/13 quarters), or 150 mg lysobactin (in 15/14 quarters), or Ubrolexin® (in 11/10 quarters) as positive control. The diseased quarters were treated with these intramammary formulations two times with an interval of 24 hours in between. Udders were clinically examined and milk samples were taken before treatment and several times after that until three weeks after the second administration. Milk was inspected visually for deviation in its consistency and samples were evaluated for presence of the challenge organism to confirm the diagnosis mastitis. Diseased udder quarters were considered microbiologically cured when *Streptococcus uberis* found in milk samples shortly before treatment could not be isolated from any milk sample taken within the time period between the seventh and the twenty-first day after the second treatment. Clinical cure was achieved when the local symptoms of mastitis completely disappeared or were at the most of very slight nature.

The microbiological cure rate on Day 7 and Day 21 after second treatment was 84.6% and 92.31% for lysobactin 50 mg, 71.4% and 85.71% for lysobactin 150 mg, and 30% and 30% for Ubrolexin®, respectively. The local clinical cure rate on Day 7 after second treatment was 86.7% both for lysobactin 50 mg and lysobactin 150 mg, and 36.4% for Ubrolexin®, respectively.

It can be concluded that lysobactin 50 and 150 mg were clearly more efficacious in comparison to the positive control product Ubrolexin® in the pivotal parameters microbiological and clinical cure.

## Claims

1. Lysobactin for use in the treatment of bovine mastitis.

2. Lysobactin for use in the treatment of bovine mastitis according to claim 1, wherein lysobactin is administered intramammarily.

3. Lysobactin for use in the treatment of bovine mastitis according to claim 1 or 2, wherein the bovine mastitis is a clinically manifest bovine mastitis.

4. Lysobactin for use in the treatment of bovine mastitis according to claim 1 or 2, wherein the bovine mastitis is a subclinical bovine mastitis.

5. Lysobactin for use in the treatment of bovine mastitis according to one of claims 1 to 4, wherein the lysobactin is provided at a dose of 25 to 1000 mg per udder quarter.

6. Lysobactin for use in the treatment of bovine mastitis according to one of claims 1 to 5, wherein the bovine mastitis is caused by *Staphylococcus* bacteria, *Streptococcus* bacteria, *Trueperella* bacteria and/or *Corynebacterium* bacteria.

7. Lysobactin for use in the treatment of bovine mastitis according to claim 6, wherein the bovine mastitis is caused by *Staphylococcus aureus,* coagulase-negative staphylococci, *Streptococcus uberis, Streptococcus dysgalacticae* and/or *Streptococcus agalacticae.*

8. Lysobactin for use in the treatment of bovine mastitis according to claim 6, wherein the bovine mastitis is caused by *Trueperella pyogenes.*

9. Lysobactin for use in the treatment of bovine mastitis according to claim 6, wherein the bovine mastitis is caused by *Corynebacterium bovis.*

## Patentansprüche

1. Lysobactin zur Verwendung bei der Behandlung von Rindermastitis.

2. Lysobactin zur Verwendung bei der Behandlung von Rindermastitis nach Anspruch 1, wobei das Lysobactin intramammär verabreicht wird.

3. Lysobactin zur Verwendung bei der Behandlung von Rindermastitis nach Anspruch 1 oder 2, wobei es sich bei der Rindermastitis um eine klinisch manifeste Rindermastitis handelt.

4. Lysobactin zur Verwendung bei der Behandlung von Rindermastitis nach Anspruch 1 oder 2, wobei es sich bei der Rindermastitis um eine subklinische Rindermastitis handelt.

5. Lysobactin zur Verwendung bei der Behandlung von Rindermastitis nach einem der Ansprüche 1 bis 4, wobei das Lysobactin in einer Dosis von 25 bis 1000 mg pro Euterviertel bereitgestellt wird.

6. Lysobactin zur Verwendung bei der Behandlung von Rindermastitis nach einem der Ansprüche 1 bis 5, wobei die Rindermastitis durch *Staphylococcus-*Bakterien, Streptococcus-Bakterien, *Trueperella-*Bakterien und/oder *Corynebacterium*-Bakterien verursacht wird.

7. Lysobactin zur Verwendung bei der Behandlung von Rindermastitis nach Anspruch 6, wobei die Rindermastitis durch *Staphylococcus aureus,* koagulasenegative Staphylokokken, *Streptococcus uberis, Streptococcus dysgalacticae* und/oder *Streptococcus agalacticae* verursacht wird.

8. Lysobactin zur Verwendung bei der Behandlung von Rindermastitis nach Anspruch 6, wobei die Rindermastitis durch *Trueperella pyogenes* verursacht wird.

9. Lysobactin zur Verwendung bei der Behandlung von Rindermastitis nach Anspruch 6, wobei die Rindermastitis durch *Corynebacterium bovis* verursacht wird.

## Revendications

1. Lysobactine pour une utilisation dans le traitement de la mastite bovine.

2. Lysobactine pour une utilisation dans le traitement de la mastite bovine selon la revendication 1, la lysobactine étant administrée par voie intra-mammaire.

3. Lysobactine pour une utilisation dans le traitement de la mastite bovine selon la revendication 1 ou 2, la mastite bovine étant une mastite bovine cliniquement manifeste.

4. Lysobactine pour une utilisation dans le traitement de la mastite bovine selon la revendication 1 ou 2, la mastite bovine étant une mastite bovine subclinique.

5. Lysobactine pour une utilisation dans le traitement de la mastite bovine selon l'une des revendications 1 à 4, la lysobactine étant fournie selon une dose allant de 25 à 1000 mg par quartier de mamelle.

6. Lysobactine pour une utilisation dans le traitement de la mastite bovine selon l'une des revendications 1 à 5, la mastite bovine étant provoquée par des bactéries de type *Staphylococcus,* des bactéries de type *Streptococcus,* des bactéries de type *Trueperella* et/ou des bactéries de type *Corynebacterium.*

7. Lysobactine pour une utilisation dans le traitement de la mastite bovine selon la revendication 6, la mastite bovine étant provoquée par *Staphylococcus aureus,* des staphylocoques à coagulase négative, *Streptococcus uberis, Streptococcus dysgalactiae* et/ou *Streptococcus agalactiae.*

8. Lysobactine pour une utilisation dans le traitement de la mastite bovine selon la revendication 6, la mastite bovine étant provoquée par *Trueperella pyogenes.*

9. Lysobactine pour une utilisation dans le traitement de la mastite bovine selon la revendication 6, la mastite bovine étant provoquée par *Corynebacterium bovis.*
